(19) Europäisches Patentamt

European Patent Office

Office européen des brevets

(11) **EP 1 506 742 A1**

(12) **EUROPEAN PATENT APPLICATION**

(43) Date of publication:
**16.02.2005 Bulletin 2005/07**

(51) Int Cl.[7]: **A61B 6/03**, G06T 5/50

(21) Application number: **04019058.9**

(22) Date of filing: **11.08.2004**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **11.08.2003 JP 2003291740**

(71) Applicant: **Hitachi, Ltd.**
**Chiyoda-ku, Tokyo 101-8010 (JP)**

(72) Inventors:
• **Kojima, Shinichi, c/o Hitachi, Ltd., Int. Prop. Gr**
**Chiyoda-ku, Tokyo 100-8220 (JP)**
• **Ueno, Yuuichirou, c/o Hitachi, Ltd., Int. Prop. Gr**
**Chiyoda-ku, Tokyo 100-8220 (JP)**

• **Amemiya, Kensuke, c/o Hitachi, Ltd., Int. Prop. Gr**
**Chiyoda-ku, Tokyo 100-8220 (JP)**
• **Yanagita, Norihito, c/o Hitachi, Ltd., Int. Prop. Gr.**
**Chiyoda-ku, Tokyo 100-8220 (JP)**
• **Kitaguchi, Hiroshi, c/o Hitachi, Ltd., Int. Prop. Gr.**
**Chiyoda-ku, Tokyo 100-8220 (JP)**
• **Tsuchiya, Katsutoshi, c/o**
**Hitachi, Ltd., Int. Prop. Gr.**
**Chiyoda-ku, Tokyo 100-8220 (JP)**
• **Yokoi, Kazuma, c/o Hitachi, Ltd., Int. Prop. Gr.**
**Chiyoda-ku, Tokyo 100-8220 (JP)**

(74) Representative: **Strehl Schübel-Hopf & Partner**
**Maximilianstrasse 54**
**80538 München (DE)**

(54) **Fusion of structural images in PET-computer tomography**

(57)     An X-ray CT image (FIG. 6A) of a low spatial resolution image is acquired by employing a PET-X-ray CT examination apparatus "B." Also, a PET image (FIG. 6B) is acquired by employing the PET-X-ray CT examination apparatus "B." Furthermore, an X-ray CT image (FIG. 6C) of a high spatial resolution image is acquired by employing another X-ray CT examination apparatus "A." Then, the X-ray CT image (FIG. 6A) equal to the low spatial resolution image is corrected by employing the X-ray CT image (FIG. 6C), so that an X-ray CT image (FIG. 6D) equal to a high spatial resolution image is obtained. Since a positional relationship of the resulting X-ray CT image (FIG. 6D) with respect to the PET image (FIG. 6B) can be grasped, this PET image (FIG. 6B) can be simply synthesized with the X-ray CT image (FIG. 6D) like an image (FIG. 6E).

## FIG.8

START

↓ S50

IMAGE X-RAY CT IMAGE BY EMPLOYING X-RAY CT EXAMINATION APPARATUS A

↓ S51

EXECUTE X-RAY CT DATA RECONSTRUCTION OF X-RAY IMAGES OF STEP S50

↓ S52

IMAGE BOTH PET IMAGE AND X-RAY CT IMAGE BY EMPLOYING PET-X-RAY CT EXAMINATION APPARATUS B

↓ S53

RECONSTRUCT PET DATA AND X-RAY CT DATA OF STEP S52

↓ S54

COMPARE X-RAY CT IMAGE OF STEP S51 WITH X-RAY XT IMAGE OF STEP S53 SO AS TO PERFORM POSITIONAL ALIGNMENT

↓ S56

CORRECT X-RAY CT IMAGE ACQUIRED IN STEP S53 BY EMPLOYING POSITIONAL RELATIONSHIP ACQUIRED IN STEP S54

↓ S57

DISPLAY SYNTHESIZED IMAGE ON MONITOR

↓

END

EP 1 506 742 A1

**Description**

BACKGROUND OF THE INVENTION

The present invention is related to a nidus position specifying system and a radiation examination apparatus with employment of an X-ray CT examination apparatus, a PET examination apparatus, a SPECT examination apparatus, and the like.

As techniques capable of imaging biological functions and biological structures of interior portions of examinees in a non-invasive manner, there are examinations using radiation. X-ray CT, MRI, PET, SPECT examination apparatuses are available as a typical radiation examination apparatus.

An X-ray CT (Computerized Tomography) imaging method corresponds to such a method that radiation emitted from an X-ray source is irradiated to an examinee, and a biological structure of an interior portion of this examinee is imaged based upon transmission of the radiation penetrated through the interior portion of the examinee. Since intensity of X-rays which have passed through the interior portion is detected by a radiation detecting element, a linear attenuation coefficient between the X-ray source and the radiation detecting element may be acquired. This linear attenuation coefficient is processed by employing a Filtered Back Projection Method described in a non-patent publication 1 so as to calculate a linear attenuation coefficient of each of voxels, and then, this calculated linear attenuation coefficient is converted into a CT value. In medical activities, a radiation source which is usually employed in an X-ray CT is approximated to maximum 140 keV, average 80 keV.

An MRI (Magnetic Resonance Imaging) method corresponds to such a method for imaging a biological structure of an interior portion of an examinee by utilizing radio waves and magnets such as a permanent magnet and an electromagnetic magnet. Elements whose proton numbers, or neutron numbers are odd numbers own inherent magnetic moment, and may induce resonant phenomena caused by external static magnetic fields. While an atom which may represent a maximum sensitivity with respect to an external magnetic field is a hydrogen atom, a large number of hydrogen molecules such as organic molecules having water and hydrogen atoms are present in an interior body of a human. Under such a circumstance, an examinee is arranged in a strong magnetic field, and then, radio waves having a specific frequency are irradiated to the examinee under such a condition that spin directions of hydrogen elements are made coincident with a constant direction. As a result, the radio waves and the hydrogen elements induce a resonant phenomenon, so that electromagnetic waves are generated. Since the electromagnetic waves are received by a coil so as to be processed by a computer, an interior distribution image of the hydrogen atoms may be acquired. This interior distribution image is referred to as an MRI image.

A PET examination corresponds to such an examination method that while a positron emitting nuclide is applied to a chemical drug and this chemical drug is injected to an examinee, a check is made that a large amount of the chemical drugs are consumed by which portion of this examinee. Positrons emitted from a chemical PET drug are coupled to electrons located in the vicinity of these positrons and then are annihilated, so that one pair of gamma rays having energy of 511 KeV are irradiated. Since these gamma rays are projected along directions completely opposite to each other, if these paired gamma rays are detected by a gamma ray detector, then it can be grasped that the positrons are emitted between which pair of detecting elements. Since the large number of these paired gamma rays are detected, it is possible to grasp such a place where a large amount of the chemical PET drugs have been consumed. Then, for example, in the case that sugar is employed as a chemical drug, it is possible to discover a cancer nidus where sugar metabolism strongly occurs. It should be noted that the acquired data is transformed to data of each voxel by using the above-described filtered back projection method, or the like.

An SPECT examination method corresponds to such a method that while a radiopharmaceutical containing a single photon emitting nuclide is injected into an examinee, a gamma ray emitted from the nuclide is detected by a gamma ray detector. Energy of a gamma ray is nearly equal to several hundreds KeV, which is emitted from such a single photon emitting nuclide which is usually employed in an SPECT examination. In the case of SPECT, since a single gamma ray is emitted, an angle of this single gamma ray entered to a detecting element cannot be acquired. As a consequence, only such a gamma ray which is entered to the detecting element from a specific angle is detected by employing a collimator, so that angular information is obtained. The SPECT examination method corresponds to such an examination method that while a radiopharmaceutical containing both a substance having a nature of being accumulated to a specific tumor and a specific molecule, and a single photon emitting nuclide ($^{99}$Tc, $^{67}$Ga, $^{201}$Tl etc.) is injected to an examinee, a gamma ray emitted from the radiopharmaceutical is detected so as to specify a place where a large amount of the radiopharmaceuticals are consumed. Also, in the case of SPECT, acquired data is transformed into data of each voxel by way of a filtered back projection method, or the like. It should also be noted that transmission images are occasionally photographed even in an SPECT. The half-value periods as to $^{99}$Tc, $^{67}$Ga, $^{201}$Tl, which are employed in the SPECT, are longer than those of radioisotopes employed in the PET, namely 6 hours up to 3 days.

Conventionally, the respective examinations such as X-ray CT and SPECT examinations have been carried out in an independent mode. However, very recently, examination apparatuses capable of continuously executing both X-ray CT examinations and PET examinations have been commercially available, for example, as described in JP-A-2003-79614 (paragraph numbers 0027 to 0028, and Fig. 1).

Also, as disclosed in JP-A-9-133771 (paragraph numbers 0011 to 0012 and Fig. 2 to Fig. 5), when a biological function image such as a PET image and a SPECT image, and either a SPECT image or a SPTCT image which are capable of judging a biological structure such as a bone scintillation are acquired at the same time, such a method for combining these images with other images has been considered.

In the present stage, there are many cases that X-ray CT examinations and PET examinations are mostly carried out in a separate manner. On the other hand, as previously explained, the examination apparatus for performing the X-ray CT examination and the PET examination in the continuous mode has been marketed, and also, the apparatus capable of simultaneously imaging both the biological structure image and the biological function image has been marketed, as described in the above-explained JP-A-2003-79614. However, depending upon the apparatus, there is a problem. That is, spatial resolution of a biological structure image is deteriorated due to a constructual aspect of this apparatus, as compared with necessary spatial resolution.

Also, there are two other problems. That is, as explained in the above-mentioned JP-A-9-133771 (paragraph numbers 0011 to 0012, and Fig. 2 to Fig. 5), when the biological function image such as the PET image and the SPECT image, and either the SPECT image or the SPTCT image which are capable of judging the biological structure such as the bone scintillation are acquired at the same time, the below-mentioned two problems may occur in the case that these acquired images are combined with other images.

As a first problem, a calculation amount becomes very large. Firstly, a biological function image is matched with a biological structure image. Next, the biological structure image which has been matched with this biological function image is matched with another biological structure image among different biological structure images from the first-mentioned matched biological structure image. Thereafter, a matching operation is carried out between the biological structure images different from the biological function images from the matching operation of these biological structure images. This calculating operation becomes very cumbersome, since the respective matching operations are normally non-linear transformation. Accordingly, there is the first problem that very lengthy time is necessarily required so as to execute the above-described calculation.

As a second problem, an amount of data is increased. That is, in such a case that a user need not require images having such spatial resolution substantially equivalent to spatial resolution of images to be combined, the image data may become redundant to therefore give pressure to a storage medium such as an HDD (hard disk drive).

SUMMARY OF THE INVENTION

The present invention has been made to solve the above-described problems of the conventional techniques, and therefore, has an object to provide both a nidus position specifying system and a radiation examination apparatus, capable of obtaining necessary spatial resolution in the case that spatial resolution of a biological structure image is deteriorated.

Since examinations based upon a plurality of modalities are carried out in most hospitals, X-ray CT data and/or MRI data as to patients are present, while these X-ray CT data and/or MRI data were acquired in the past, or have been acquired in separate manners. Also, in the case of the same modality, positional relationships among these medical data can be very easily grasped. While an attention has been paid to these technical points, according to the present invention, an X-ray CT image and an MRI image which have been simultaneously imaged are corrected by employing such an information acquired in X-ray CT and MRI examinations as to an examinee, which have been executed in a separate manner. In other words, since the same modality is employed, if a positional alignment is surely carried out, then the presently acquired information can be readily corrected by employing the past-acquired information.

Moreover, another object of the present invention is to provide a method capable of executing a positional aligning operation of images in a higher speed as well as in a higher function. For instance, as explained in the above-mentioned JP-A-9-133771 (paragraph numbers 0011 to 0012, and Fig. 2 to Fig. 5), when the biological function image such as the PET image and the SPECT image, and either the SPCT image or the SPTCH image which are capable of judging the biological structure such as the bone scintillation are acquired at the same time, there is the method for combining the acquired images with other images. However, this method owns the above-explained two problems.

As a consequence, a positional aligning method of images has also be invented in accordance with a technical idea of the present invention. In a positional alignment of images in accordance with the present invention, while images which have been acquired either at the same time or in a continuous mode are employed as reference images, the positional aligning operation is carried out. That is to say, such a mapping transformation from a biological function image into another biological structure image which has been separately imaged is not required in this inventive positional alignment. Instead of this mapping transformation, according to the present invention, a positional aligning operation is carried out based upon both a stage for executing an image synthesizing operation between a biological function image and a biological structure image which has been acquired at the same time, and also, another stage for correcting the biological structure images by way of an interpolation work. This positional aligning method owns two merits. As a first merit, since there is no need to match the biological structure images different from the biological function image, such a cumbersome calculation for coupling the non-linear transformations with each other is no longer required. Furthermore, in the stage of the interpolation work between the biological structural images, the interpolation portion between the biological structure images, and the spatial resolution of the interpolated image can be changed. In other words, in such a case that an interpolating operation is carried out by employing a high precision image and a low precision image, such an image having intermediate spatial resolution may also be acquired. Moreover, this interpolating operation may be applied to an entire image, and also, to only a partial image thereof. This may cause a merit capable of reducing an image size.

Next, a description is made of a simple sequential operation, assuming now that a biological structure image, an image acquired by simultaneously imaging a biological function image, and another biological structure image which has been separately imaged are obtained. In this case, it is so assumed that while the biological structure image corresponds to an X-ray CT image and the biological function image corresponds to a PET image, both a high resolution X-ray CT examination apparatus and a simultaneous PET-X-ray CT imaging apparatus are provided; and both simultaneous imaging results of X-ray CT and PET imaging operations, and a high resolution X-ray CT image which has been separately images are acquired.

First, an X-ray CT image acquired from the X-ray CT examination apparatus is compared with another X-ray CT image acquired from the simultaneous X-ray CT-PET imaging apparatus by a computer designed for an X-ray CT image positional alignment in order to clarify a positional relationship between both the X-ray CT images. At this time, a non-linear transformation is carried out, if required. Next, the simultaneously-imaged X-ray CT image is corrected by employing the high resolution X-ray CT image. This correction may be realized in such a manner that data as to such a portion required for the voxel size of the high resolution X-ray CT image is transformed by employing the previously acquired positional relationship. Finally, both the X-ray CT corrected image and the PET image which have been acquired are displayed on a display unit. At this time, while the positional relationship which is known by executing the simultaneous imaging operations is used, both the PET image and the X-ray CT image may be alternatively displayed in an overlapping mode, or may be alternatively arrayed side by side from which the positional relationship between these images may be grasped.

In accordance with the present invention, the precision of the biological structure image which is required so as to grasp a nidus position of the biological function image can be obtained based upon a small amount of data, so that a precise position of a region of interest as to the biological function image can be readily specified.

Other objects, features and advantages of the invention will become apparent from the following description of the embodiments of the invention taken in conjunction with the accompanying drawings.

BRIEF DESCRIPTION OF THE DRAWINGS

Fig. 1 is a cross-sectional view for schematically showing an X-ray CT examination apparatus according to a first embodiment of the present invention.
Fig. 2 is a lateral-sectional view of schematically indicating the X-ray CT examination apparatus shown in Fig. 1.
Fig. 3 is a cross-sectional view for schematically representing a PET-X-ray CT examination apparatus according to the first embodiment of the present invention.
Fig. 4 is a lateral-sectional view for schematically showing the PET-X-ray CT examination apparatus indicated in Fig. 3.
Fig. 5 is an internal structural diagram for indicating a signal discriminating apparatus shown in Fig. 3, which has been enlarged.

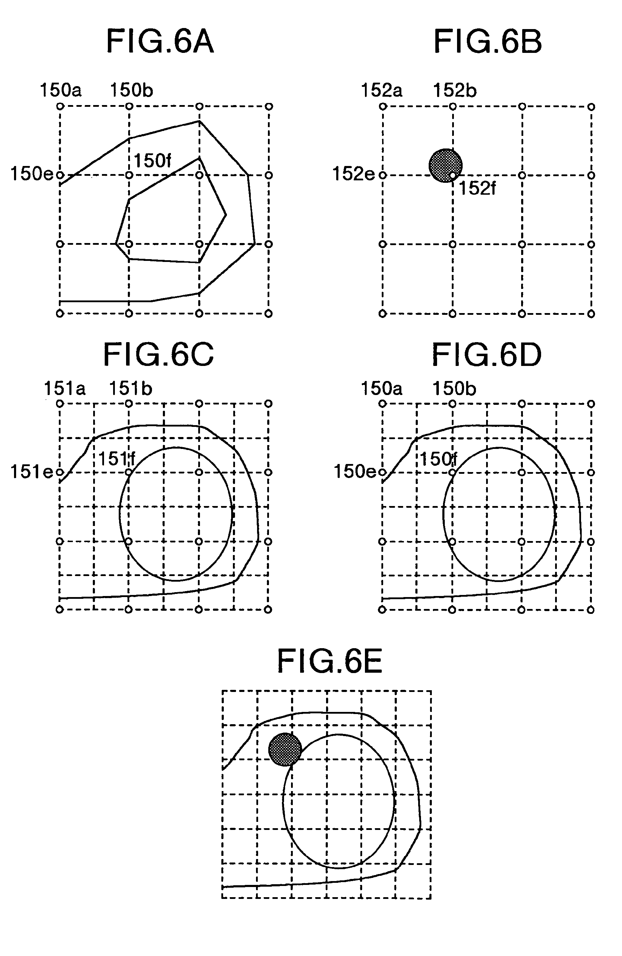

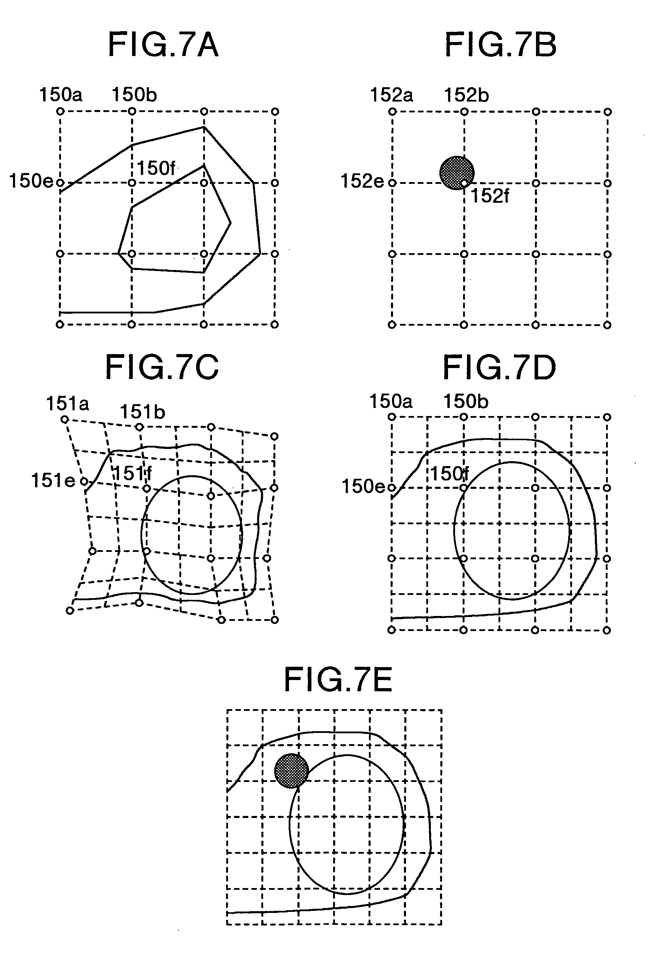

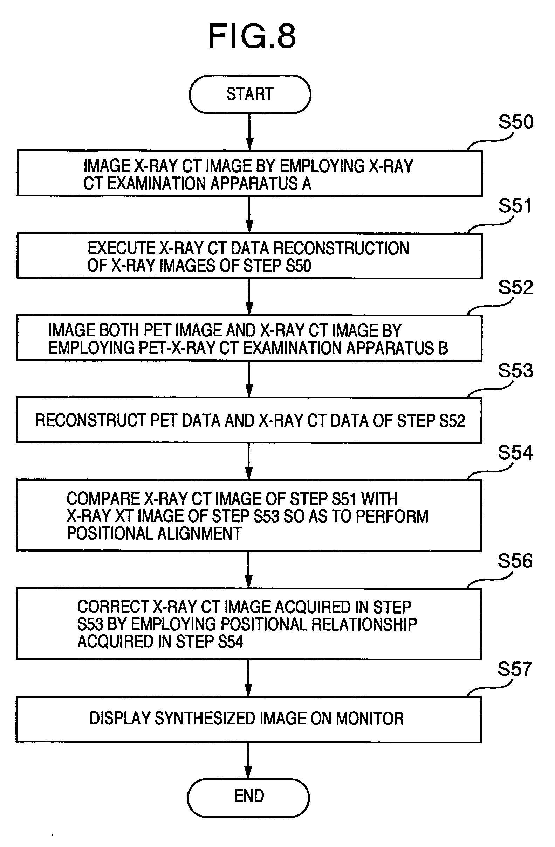

Figs. 6A-6E are explanatory diagrams for showing one image interpolating method executed in a step S56 in a flow chart of Fig. 8; Fig. 6A is a diagram for showing an X-ray CT image of a low spatial resolution image acquired by the PET-X-ray CT examination apparatus; Fig. 6B is a diagram for representing a PET image acquired by the PET-X-ray CT examination apparatus; Fig. 6C is a diagram for indicating an X-ray CT image of a high spatial resolution image acquired by the X-ray CT examination apparatus; Fig. 6D is a diagram for denoting that the X-ray CT image of Fig. 6A is corrected by employing the X-ray CT image of Fig. 6C; and Fig. 6E is a diagram for showing that the PET image of Fig. 6B is synthesized by the X-ray CT image of Fig. 6D; Figs. 7A-7E are explanatory diagrams for showing another image interpolating method executed in the step S56 in the flow chart of Fig. 8; Fig. 7A is a diagram for showing an X-ray CT image of a low spatial resolution image acquired by the PET-X-ray CT examination apparatus; Fig. 7B is a diagram for representing a PET image acquired by the PET-X-ray CT examination apparatus; Fig. 7C is a diagram for indicating an X-ray CT image of a high spatial resolution image acquired by the X-ray CT examination apparatus; Fig. 7D is a diagram for denoting that the X-ray CT image of Fig. 7A is corrected by employing the X-ray CT image of Fig. 7C; and Fig. 7E is a diagram for showing that the PET image of Fig. 7B is synthesized by the X-ray CT image of Fig. 7D; Fig. 8 is a flow chart for describing imaging sequential operations executed by both the X-ray CT examination apparatus and the PET-X-ray CT examination apparatus, according to the first embodiment of the present invention.

Fig. 9 is a perspective view for schematically showing a parallel PET-X-ray CT examination apparatus according to a second embodiment of the present invention.

Fig. 10 is a cross-sectional view for representing the PET examination apparatus shown in Fig. 9.

Fig. 11 is a flow chart for describing imaging sequential operations executed by an X-ray CT examination apparatus and the parallel PET-X-ray CT examination apparatus, according to the second embodiment of the present invention.

Fig. 12 is a flow chart for describing imaging sequential operations executed by an X-ray CT examination apparatus and a PET-X-ray CT examination apparatus, according to a third embodiment of the present invention.

Fig. 13 is a flow chart for describing imaging sequential operations executed by an X-ray CT examination apparatus and a PET-X-ray CT examination apparatus, according to a fourth embodiment of the present invention.

DETAILED DESCRIPTION OF THE EMBODIMENTS

(FIRST EMBODIMENT)

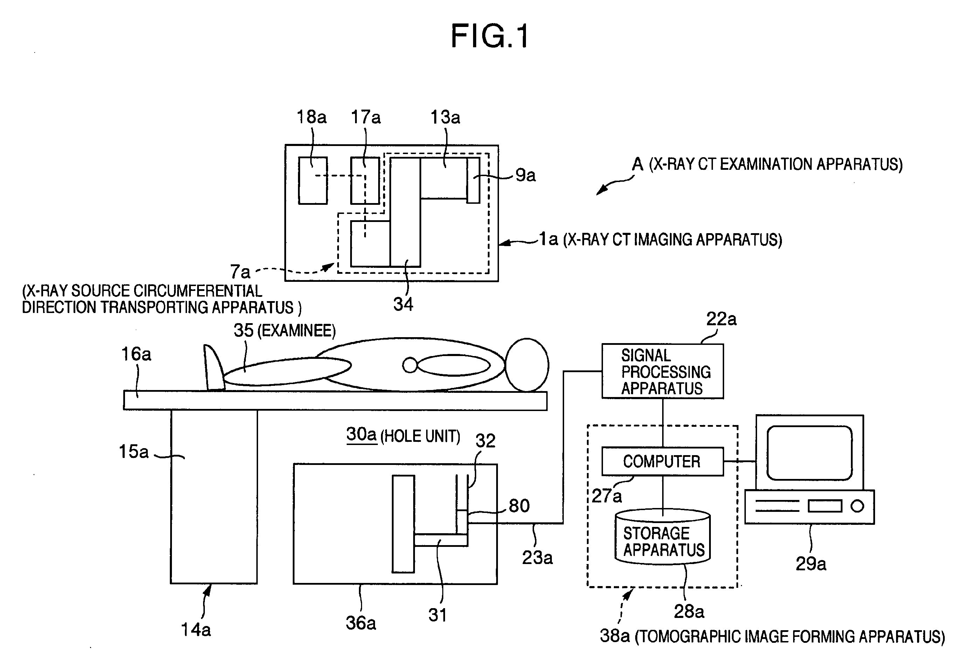

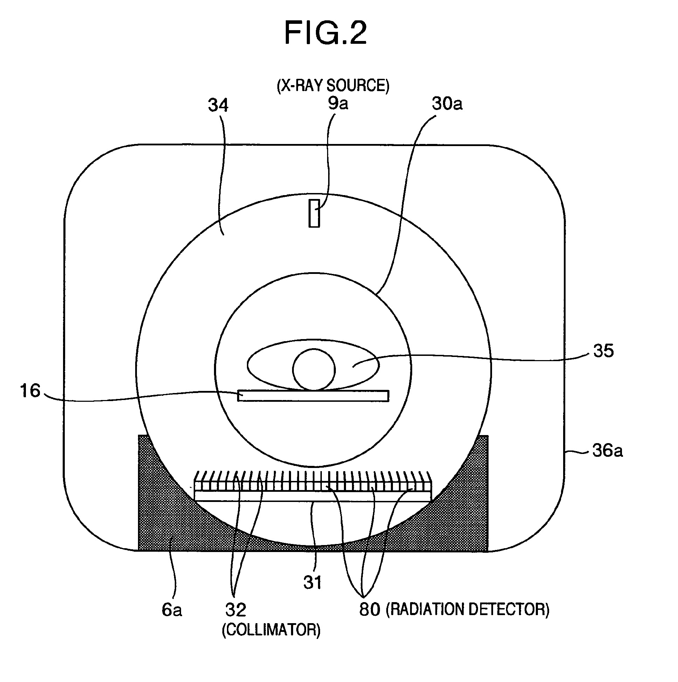

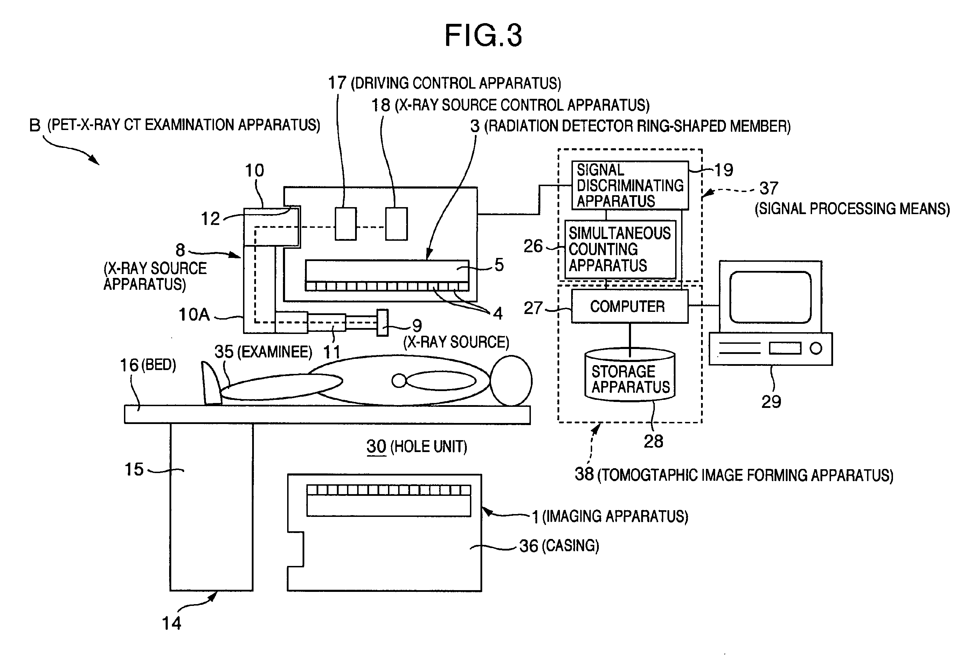

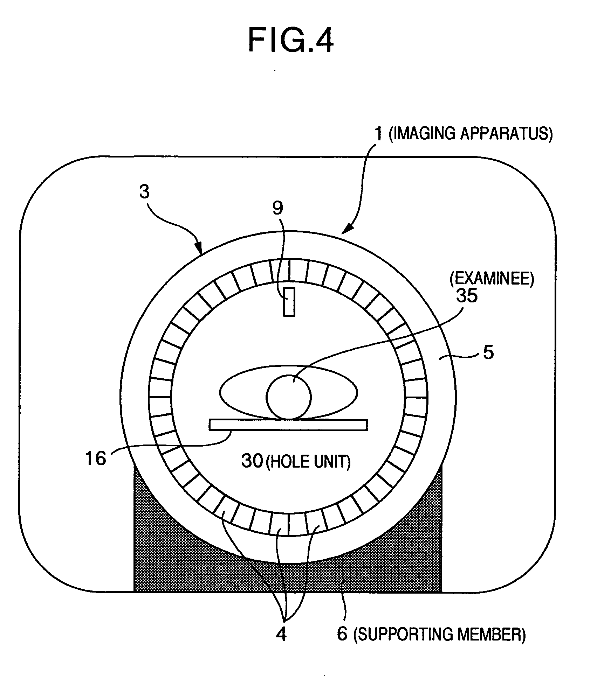

A first embodiment of the present invention will now be described with reference to Fig. 1 to Fig. 8. Fig. 1 is an X-ray CT examination apparatus, and Fig. 2 is a sectional view of the X-ray CT examination apparatus. Fig. 3 and Fig. 4 represent PET-X-ray CT examination apparatus.

In this first embodiment, a nidus position specifying system for specifying a nidus position of an examinee 35 is constituted by a PET-X-ray CT examination apparatus "B" (see Fig. 3) and an X-ray CT examination apparatus "A" (see Fig. 1). The PET-X-ray CT examination apparatus B corresponds to a biological function examination apparatus and a first biological structure examination apparatus. The X-ray CT examination apparatus A corresponds to a second biological structure examination apparatus.

First, as shown in Fig. 1, the X-ray CT examination apparatus A, according to this first embodiment, contains an X-ray CT imaging apparatus 1a, an examinee holding apparatus 14a, a tomographic image forming apparatus 38a, and a display apparatus 29a. The tomographic image forming apparatus 38a contains a computer 27a (for example, workstation), and a storage apparatus 28a. The examinee holding apparatus 14a contains a supporting member 15a, and a bed 16a which is mounted on an upper edge portion of the supporting member 15a in such a manner that the bed 16a can be moved along a longitudinal direction thereof. The examinee 35 is laid on the bed 16a.

While the X-ray CT imaging apparatus 1a is installed along a direction perpendicular to the longitudinal direction of the bed 16a, this X-ray CT imaging apparatus 1a contains a radiation detector 80 (see Fig. 2), an X-ray source circumferential direction transporting apparatus 7a, a driving apparatus control apparatus 17a, an X-ray source control apparatus 18a, and a casing 36a (see Fig. 2). Also, the X-ray source circumferential direction transporting apparatus 7a is equipped with a disk-shaped holding apparatus 34, an X-ray source 9a, and an X-ray source apparatus holding unit 13a. The X-ray source apparatus holding unit 13a is mounted on an outer surface of the disk-shaped holding unit 34 at one edge portion of the disk-shaped holding unit 34. The X-ray source 9a is mounted on the other edge portion of the X-ray source apparatus holding unit 13a.

The radiation detector 80, the X-ray source circumferential direction transporting apparatus 7a, the driving apparatus control apparatus 17a, and the X-ray source control apparatus 18a are installed in the casing 36a. The radiation detector 80 is arranged at a position where the radiation detector 80 can detect X-ray which has penetrated from the X-ray source 9a through the examinee 35. A plurality (approximately 100 pieces) of radiation detectors 80 are arrayed from the disk-shaped holding unit 34 via the detector holding unit 31, and are rotated around both the examinee holding apparatus 14a and the examinee 35 in conjunction with the X-ray source circumferential direction transporting apparatus 7a. A collimator 32 is mounted on the radiation detectors 80. Only X-rays generated from the X-ray source 9a are entered into the radiation detectors 80. The radiation detector 80 corresponds to a scintillation detector.

The X-ray source 9a contains an X-ray tube which is not shown in the drawing, and is known in the field. As to this X-ray tube, an anode, a cathode, a current source of the cathode, and a voltage source which applies a voltage between the anode and the cathode are provided in an outer tube thereof. The cathode corresponds to a filament made of tungsten. Since a current is supplied from the current source to the cathode, electrons are emitted from the cathode. The electrons are accelerated by a voltage (140 KV) which is applied from the voltage source between the cathode and the anode, and then, the accelerated electrons impinge on the anode (made of W, Mo, and the like) corresponding to a target. X-rays lower than, or equal to 140 KeV are generated by bombardments to the anode, and then, are emitted from the X-ray source 9a.

The X-ray source control apparatus 18a controls emission time of X-rays emitted from the X-ray source 9a. In other words, the X-ray source control apparatus 18a repeatedly carries out such a control operation that during an X-ray CT examination, this X-ray source control apparatus 18a outputs an X-ray generating signal so as to close a switching device (will be referred to as "X-ray source switching device" hereinafter, and is not shown in this drawing) which is provided between the power supply and the anode (otherwise cathode) of the X-ray tube employed in the X-ray source 9a; when a first setting time has elapsed, the X-ray source control apparatus 18a outputs an X-ray stop signal so as to open the X-ray source switching device; and then, when a second setting time has elapsed, the X-ray source control apparatus 18a closes the X-ray source switching device. The voltage is applied between the anode and the cathode during the first setting time, whereas no voltage is applied between them during the second setting time. Since this control operation is carried out, the X-rays are emitted in a pulse shape from the X-ray tube.

When an X-ray CT examination is commenced, the driving apparatus control apparatus 17a outputs a drive starting signal so as to close a switching device (will be referred to as "first motor switching device" hereinafter) which is connected to the power supply. Since a current is supplied, a first motor is rotated, rotation force of this first motor is transferred via a driving force transferring mechanism to a pinion, and thus, this pinion is rotated. Since the pinion is rotated, the disk-shaped holding unit 34, namely the X-ray source 9a is transported around the examinee 35 at a setting speed. When the X-ray CT examination is ended, the driving apparatus control apparatus 17a outputs a drive stop signal so as to open the first motor switching device. As a result, the transporting operation of the X-ray source 9a along the circumferential direction is stopped. Also, since the radiation detectors 80 are also fixed via the detector holding unit 31 to the disk-shaped holding unit 34, these radiation detectors 80 are rotated in combination with the X-ray source 9a. As a consequence, when the X-rays are irradiated from the X-ray source 9a, it is so arranged that X-rays which have penetrated the examinee 35 can be measured by the radiation detectors 80. The supporting member 6a is installed on a floor of an examination room.

When the X-ray CT examination is commenced, the drive starting signal outputted from the drive apparatus control apparatus 17a is entered to the X-ray source control apparatus 18a. In response to the input of the drive starting signal, the X-ray source control apparatus 18a outputs an X-ray generating signal. Thereafter, the X-ray source control apparatus 18a repeatedly outputs both the X-ray stop signal and the X-ray generating signal. Since the X-ray stop signal and the X-ray generating signal are repeatedly outputted, the X-ray source 9a emits X-rays during a setting time (for example, 1 μsec.), and thereafter, stops the emission of the X-rays. Both the emitting operation and the stopping operation of the X-rays are repeatedly carried out during transporting time period of the X-ray source 9a along the circumferential direction. The X-rays emitted from the X-ray source 9a are irradiated to the examinee 35 located in a hole unit 30a in a fan-beam shape. Since the X-ray source 9a is transported along the circumferential direction, the examinee 35 on the bed 16 is irradiated by the X-rays from the peripheral portion thereof. The X-rays which has passed through the examinee 35 are detected by the radiation detector 80 which is located at a position opposite to the X-ray source 9a at an angle of 180 degrees along the opposite direction while an axial center of the hole unit 30a is set as a base point, and is rotated in a similar manner to the X-ray source 9a. These radiation detectors 80 output detection signals of the X-rays (will be referred to as "X-ray detection signals" hereinafter). The X-ray detection signals are transferred via relevant wiring lines 23a to the signal processing apparatus 22a corresponding thereto. The signal processing apparatus 22a calculates intensity of the X-ray detection signals based upon the entered X-ray detection signals, and then, outputs the intensity information. The intensity information of the X-ray detection signals outputted from the signal processing apparatus 22a is stored via the computer 27a to the storage apparatus 28a.

Next, a description is made of a method for reconstructing images of X-ray CT data which have been acquired by the X-ray CT examining apparatus A constructed as explained above. As the image reconstructing method of the X-ray CT data, for instance, the image reconstructing operation is carried out by employing the Filtered Back Projection Method which is described in IEEE Transaction on Nuclear Science, NS-21 volume, page 21. An acquired image corresponds to a CT value in either a rectangular parallelepiped voxel or a cube voxel, in which an interior body is segmented in an equi-interval along an x direction, a y direction, and a z direction, respectively. The CT value may be expressed by the following equation (1), while employing a linear attenuation coefficient "μ" of each of the positions, and a linear attenuation coefficient "μw":

$$\text{CT value} = k(\mu - \mu w)/\mu w \quad \text{(equation 1)}.$$

In this equation, as a value of "k", either 500 or 1000 is employed. In this description, in such a case that the CT value is observed, it is so set that k=1000.

Next, a PET-X-ray CT examination apparatus B corresponding to the radiation examination apparatus will now be explained with reference to Fig. 3 to Fig. 5. The PET-X-ray CT examination apparatus B is equipped with an imaging apparatus 1, an examinee holding apparatus 14, a signal processing apparatus 37, a tomographic image forming apparatus 38, and a display apparatus 29. The signal processing apparatus 37 contains a signal discriminating apparatus 19 and a simultaneous counting apparatus 26. The tomographic image forming apparatus 38 contains a computer 27 (for instance, workstation) and a storage apparatus 28. The examinee holding apparatus 14 contains a supporting member 15, and a bed 16 which is mounted on an upper edge portion of the supporting member 15 in such a manner that the bed 16 can be moved along a longitudinal direction thereof.

While the imaging apparatus 1 is installed along a direction perpendicular to the longitudinal direction of the bed 16, this imaging apparatus 1 contains a radiation detector ring-shaped member 3, an X-ray source circumferential direction transporting apparatus 7, a driving apparatus control apparatus 17, an X-ray source control apparatus 18, and a casing 36. The radiation detector ring-shaped member 3, the X-ray source circumferential direction transporting apparatus 7, the driving apparatus control apparatus 17, and the X-ray source control apparatus 18 are installed inside the casing 36. The radiation detector ring-shaped member 3 contains a ring-shaped holding unit 5, and a large number of radiation detectors 4 which are installed in a ring shape inside the ring-shaped holding unit 5. A hole unit 30 is formed inside the radiation detectors 4 of the radiation detector ring-shaped member 3. The hole unit 30 is made of a through hole, and corresponds to an observation region into which the bed 16 is inserted. A large number of radiation detectors 4 (approximately 10,000 pieces in total) are arranged in the form of plural arrays in such a manner that these radiation detectors 4 are installed on the ring-shaped holding unit 5 not only along the circumferential direction, but also along the axial direction of the hole unit 30. The radiation detector 4 corresponds to a semiconductor radiation detector, while a semiconductor element portion thereof is constructed of CdTe (cadmium tellurium) which is made of a cube sized by 5 mm X 5 mm and corresponds to a detecting portion thereof. Alternatively, this element portion may be constituted by either GaAs (gallium arsenide) or CZT (cadmium zinc tellurium). The ring-shaped holding unit 5 is installed on the supporting member 6. The supporting members 6 and 15 are installed on a floor of an examination room. Both the driving apparatus control apparatus 17 and the X-ray source control apparatus 18 are installed inside the casing 36 in combination with the ring-shaped holding unit 5. A guide rail 12 is formed in the form of a concave in one edge surface of the casing 36. The guide rail 12 has a ring shape and surrounds a peripheral portion of the hole unit 30. The X-ray source apparatus 8 contains an X-ray source 9, the X-ray source driving apparatus 10, and an axial direction transporting arm 11.

The X-ray source driving apparatus 10 is equipped with a first motor and a power transferring mechanism (none of them is shown) within the casing 10A. The power transferring mechanism is coupled to a rotation shaft of the first motor. The telescopically-drivable axial direction transporting arm 11 is mounted on the casing 10A of the X-ray source driving apparatus 10, and is extended to the inside portion of the hole unit 30. The X-ray source 9 is mounted on the axial direction transporting arm 11. The axial direction transporting arm 11 is telescopically driven by a second motor (not shown) installed in the X-ray source driving apparatus 10. The X-ray source driving apparatus 10 is mounted on the guide rail 12 in such a manner that this X-ray source driving apparatus 10 is not dropped, but can be transported along the guide rail 12. Although not shown in the drawing, the X-ray source driving apparatus 10 owns a pinion. This pinion is meshed with a rack provided on the guide rail 12 so as to receive rotation force from the power transferring mechanism.

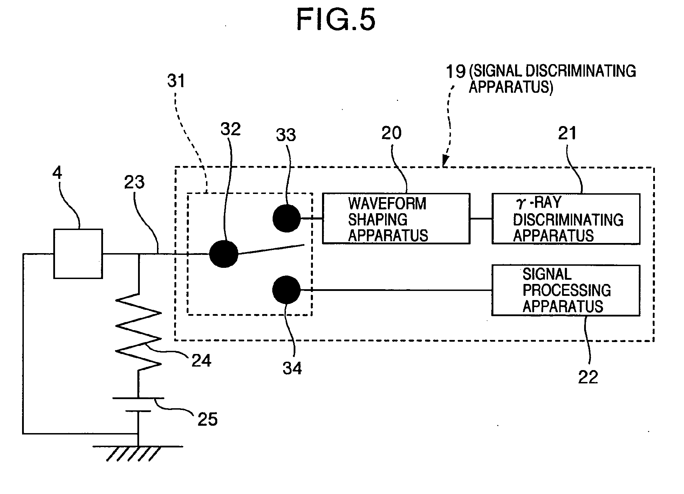

Although not shown in the drawing, the X-ray source 9 contains an X-ray tube which is known in this field. The respective radiation detectors 4 are connected by respective wiring lines 23 to movable terminals 32 (see Fig. 5) of the corresponding signal discriminating apparatus 19. The signal discriminating apparatus 19 are provided with the respective radiation detectors 4 one by one. A detailed structure of one signal discriminating apparatus 19 is indicated in Fig. 5. This signal discriminating apparatus 19 is equipped with a switch 31, a waveform shaping apparatus 20, a $\gamma$-ray discriminating apparatus 21, and a signal processing apparatus 22 for acquiring intensity of X-rays. The switch 31 contains the movable terminal 32, and two fixed terminals 33 and 34. The waveform shaping apparatus 20 is connected to both the fixed terminal 33 and the $\gamma$-ray discriminating apparatus 21. The signal processing apparatus 22 is connected to the fixed terminal 34. A power supply 25 is connected to the wiring line 23 and the radiation detector 4. The $\gamma$-ray discriminating apparatus 21 is connected via a simultaneous counting apparatus 26 to a computer 27. A total number of the simultaneous counting apparatus 26 is 1, and is connected to all of the $\gamma$-ray discriminating apparatuses 21. Each of the signal processing apparatus 22 is connected to the computer 27. Both the storage apparatus 28 and the display apparatus 29 are connected to the computer 27. The signal discriminating apparatus 19 is equipped with an X-ray detection signal processing apparatus containing the signal processing apparatus 22, and a $\gamma$-ray detection signal processing apparatus containing both the waveform shaping apparatus 20 and the $\gamma$-ray discriminating apparatus 21.

A concrete X-ray CT examination and a concrete PET examination, which are executed by the imaging apparatus 1, will now be described. First, a chemical PET drug is injected into the examinee 35 in order that injected body interior radiation becomes equal to 370 MBq. The chemical PET drug is selected in accordance with a purpose of examination (place of cancer is grasped, cardiac arteria stream is examined, etc.). After the chemical PET drug has been injected and a predetermine time period has elapsed, while the examinee 35 is laid on the bed 16, this examinee 35 is inserted into the hole unit 30.

The X-ray source control apparatus 18 controls emission time of X-rays emitted from the X-ray source 9. In other words, the X-ray source control apparatus 18 repeatedly carries out such a control operation that during an X-ray CT examination, this X-ray source control apparatus 18 outputs an X-ray generating signal so as to close a switching device (will be referred to as "X-ray source switching device" hereinafter, and is in drawing) which is provided between the power supply and the anode (otherwise cathode) of the X-ray tube employed in the X-ray source 9; when a first setting time has elapsed, the X-ray source control apparatus 18 outputs an X-ray stop signal so as to open the X-ray source switching device; and then, when a second setting time has elapsed, the X-ray source control apparatus 18 closes the X-ray source switching device. The voltage is applied between the anode and the cathode during the first setting time, whereas no voltage is applied between them during the second setting time. Since this control operation is carried out, the X-rays are emitted in a pulse shape from the X-ray tube. An irradiation time "T" corresponding to the first setting time is set to be, for example, 1 μsec in order that detection probability of γ rays (gamma rays) on the radiation detectors 4 can be neglected. The second setting time corresponds to such a time that the X-ray source 9 is moved between one radiation detector 4 and another radiation detector 4 which is located adjacent to the first-mentioned radiation detector 4 along the circumferential direction. This second setting time is determined based upon a moving speed of the X-ray source 9 along the circumferential direction of the guide rail 12. Both the first setting time and the second setting time have been stored in the X-ray source control apparatus 18.

When an X-ray CT examination is commenced, the driving apparatus control apparatus 17 outputs a drive starting signal so as to close a switching device which is connected to the first motor of the X-ray source driving apparatus 10 so a to be connected to the power supply. Since a current is supplied, the first motor is rotated, rotation force of this first motor is transferred via a driving force transferring mechanism to a pinion, and thus, this pinion is rotated. Since the pinion is rotated, the X-ray source apparatus 8, namely the X-ray source 9 is transported around the peripheral area of the examinee 35 at a setting speed along the guide rail 12. When the X-ray CT examination is ended, the driving apparatus control apparatus 17 outputs a drive stop signal so as to open the above-described switching device. As a result, the transporting operation of the X-ray source 9 along the circumferential direction is stopped.

In this first embodiment, all of the radiation detectors 4 which are arranged in the ring shape along the circumferential direction are not moved along the circumferential direction thereof, but also, are not moved along the axial direction of the hole unit 30. In order that the control signal is transferred from both the X-ray source control apparatus and the driving apparatus control apparatus, which are not moved, to the X-ray source apparatus which is moved, the technical idea which is known in the field is applied. This technical idea never disturbs the transportation of the X-ray source apparatus.

When the X-ray CT examination is commenced, a drive starting signal outputted from the drive apparatus control apparatus 17 is entered to the X-ray source control apparatus 18. In response to the input of the drive starting signal, the X-ray source control apparatus 18 outputs an X-ray generating signal. Thereafter, the X-ray source control apparatus 18 repeatedly outputs both the X-ray stop signal and the X-ray generating signal. Since the X-ray stop signal and the X-ray generating signal are repeatedly outputted, the X-ray source 9 emits X-rays during the first setting time (for example, 1 μsec.), and thereafter, stops the emission of the X-rays during the second setting time. Both the emitting operation and the stopping operation of the X-rays are repeatedly carried out during transporting time period of the X-ray source 9 along the circumferential direction. The X-rays emitted from the X-ray source 9 are irradiated to the examinee 35 located in the hole unit 30 in a fan-beam shape. Since the X-ray source 9 is transported along the circumferential direction, the examinee 35 on the bed 16 is irradiated by the X-rays from the peripheral portion thereof. The X-rays which has passed through the examinee 35 are detected by a plurality of radiation detectors 4 which are located at positions opposite to the X-ray source 9 at an angle of 180 degrees along the opposite direction while these plural radiation detectors 4 are positioned at a center in the circumferential direction. These radiation detectors 4 output detection signals of the X-rays (will be referred to as "X-ray detection signals" hereinafter). The X-ray detection signals are transferred via relevant wiring lines 23a to the signal discriminating apparatuses 19 corresponding thereto. It should be noted that these radiation detectors 4 which detect the above-explained X-rays will be referred to as "first radiation detectors 4" for the sake of convenience.

Gamma (γ) rays of 511 KeV which are caused by the chemical PET drug are being emitted from the examinee 35 located inside the hole unit 30. The radiation detectors 4 other than the first radiation detectors 4 detect the γ rays, and then, outputs detection signals of these γ rays (will be referred to as "γ-rays detection signals" hereinafter). The γ-rays detection signals are entered via the relevant wiring lines 23 to the respective signals discriminating apparatus 19. Such radiation detectors 4 which are detecting the γ rays will be referred to as "second radiation detectors 4" for the sake of convenience.

The γ-rays detection signals outputted from the second radiation detectors 4 are transferred to the γ-rays discriminating apparatus 21, and the X-ray detection signals outputted from the first radiation detectors 4 are transferred to the signal processing apparatus 22 within the signal discriminating apparatus 19. Such a transfer operation of the respective detection signals is carried out by selectively switching the switch 31 of the signal discriminating apparatus 19. A switching operation for connecting the movable terminal 32 of the switch 31 to either the fixed terminal 33 or the fixed terminal 34 is carried out in response to a switching control signal which corresponds to the output signal of the driving apparatus control apparatus 17. As previously explained, the driving apparatus control apparatus 17 controls the transporting operation of the X-ray source apparatus 10. At the same time, this driving apparatus control apparatus 17 selects the first radiation detectors 4, and connects the movable terminal 32 of the switch 31 to the fixed terminal 34 thereof in the signal discriminating apparatus 19 which is connected to this first radiation detector 4.

A description is made of a selecting operation as to the first radiation detectors 4. An encoder (not shown) is coupled to the first motor provided in the X-ray source driving apparatus 10. The driving apparatus control apparatus 17 inputs thereinto a detection signal of the encoder so as to acquire a position of the X-ray source driving apparatus 10, namely, a position of the X-ray source 9 along the circumferential direction, and selects such a radiation detector 4 which is located at a position opposite to this acquired position of the X-ray source 9 at an angle of 180 degrees by employing the positional data of the respective radiation detectors 4 which have been stored. Since X-rays emitted from the X-ray source 9 own certain widths in the circumferential direction of the guide rail 12, there are provided plural pieces of radiation detectors 4 for detecting such X-rays which have penetrated through an interior portion of the examinee 35 along the circumferential direction other than this selected radiation detector 4. The driving apparatus control apparatus 17 also selects these plural pieces of the radiation detectors 4. These radiation detectors 4 correspond to the first radiation detectors. The first radiation detectors 4 are also different from each other in connection with the transportation of the X-ray source 9 along the circumferential direction. It is so observed that the first radiation detectors 4 are also moved along the circumferential direction in a quasi-manner in connection with the transportation of the X-ray source 9 along the circumferential direction. When the driving apparatus control apparatus 17 selects other radiation detectors 4 in connection with the transportation of the X-ray source 9 along the circumferential direction, movable terminals 32 connected to such radiation detectors 4 which newly become the first radiation detectors 4 are connected to the fixed terminals 34. The movable terminals 32 which are connected to the radiation detectors 4 which have not become the first radiation detectors 4 in connection with the transportation of the X-ray source 9 along the circumferential direction are connected to the fixed terminals 33 by the driving apparatus control apparatus 17.

It is also possible to say that the first radiation detectors 4 may correspond to such radiation detectors 4 which are connected to the signal processing apparatus 22 by the switch 31. Further, it is also possible to say that the second radiation detectors 4 may correspond to such radiation detectors 4 which are connected to the γ-ray discriminating apparatus 21 by the switch 31. The respective radiation detectors 4 which are installed in the ring-shaped holding unit 5 may constitute the first radiation detectors 4 in one case, and may constitute the second radiation detectors 4 in another case, depending upon a positional relationship with respect to the X-ray source 9. As a consequence, one radiation detector 4 may output both an X-ray detection signal and a γ-ray detection signal, although these X-ray detection signal and γ-ray detection signal are temporally separately outputted.

The first radiation detectors 4 detect such X-rays which have been irradiated from the X-ray source 9 and then have penetrated through the examinee 35 during 1 μsec equal to the first setting time. The probability is negligibly small at which the first radiation detectors 4 detect γ rays emitted from the examinee 35 during 1 μsec. A large number of γ rays which have been generated within the interior portion of the examinee 35 due to the chemical PET drug are not emitted along a specific direction, but are emitted along a universal direction. As previously explained, these γ rays are emitted in such a condition that these γ rays constitute paired γ rays, and the paired γ rays are emitted along directions substantially opposite to each other (180 degrees ± 0.6 degrees), and then, are detected by way of the second radiation detectors 4 of the radiation detector ring-shaped member 4.

A description is made of a signal process operation executed by the signal discriminating apparatus 19 when both an X-ray detection signal and a γ-ray detection signal which are outputted from a radiation detector 4 are entered into this signal discriminating apparatus 19. As previously explained, the X-ray detection signal outputted from the first radiation detector 4 is inputted into the signal processing apparatus 22 by way of the switch 31. The signal processing apparatus 22 calculates intensity of the X-ray detection signal based upon the inputted X-ray detection signal, and then, outputs this calculated intensity information.

The γ-ray detection signal outputted from the second radiation detector 4 is inputted to the waveform shaping apparatus 20 by way of the switch 31. The waveform shaping apparatus 20 converts the inputted γ-ray detection signal into such a γ-ray detection signal having a Gaussian distribution waveform, and then, outputs this converted γ-ray detection signal. As previously explained, energy of such γ rays is equal to 511 KeV, which are produced in the interior body of the examinee 35, since positrons emitted from the chemical PET drug are annihilated. However, all of the energy of the γ rays within the semiconductor element portion are not always changed into electrons. On the other hand, in order to eliminate noise which is caused by low energy gamma rays which have been scattered in the interior body of the examinee 35, only such γ-ray detection signals which are approximated to 511 KeV are required as data. As a consequence, while the γ-ray discriminating apparatus 21 defines energy of e.g., 450 KeV lower than 511 KeV as an energy setting value, when an imaging signal having energy higher than, equal to this energy setting value (will be referred to as "first energy setting value") is inputted into the γ-ray discriminating apparatus 21, this γ-ray discriminating apparatus 21 generates a pulse signal having predetermined energy. In other words, the γ-ray discriminating apparatus 21 corresponds to such an apparatus for generating the pulse signal having the above-described energy when such an imaging signal (namely, γ-ray detection signal) having energy higher than, or equal to the first energy setting value is inputted thereinto.

As previously explained, in order that a γ-ray detection signal having specific energy is processed in the γ-ray discriminating apparatus 21, such a filter capable of penetrating therethrough imaging signals having a predetermined energy range may be alternatively provided within the γ-ray discriminating apparatus 21 (otherwise, prestage of γ-ray discriminating apparatus 21). The γ-ray discriminating apparatus 21 generates a pulse signal with respect to an imaging signal which has passed a first filter.

The simultaneous counting apparatus 26 performs a simultaneous counting operation every γ-ray pair in such a manner that the pulse signals outputted from the γ-ray discriminating apparatuses 21 of the respective signal discriminating apparatuses 19 are entered into this simultaneous counting apparatus 26, and these inputted pulse signals are employed, and then, calculate a count value with respect to the γ-ray detection signal. Furthermore, the simultaneous counting apparatus 26 processes two detection points where each of γ rays of a γ-ray pair have been detected based upon one pair of pulse signals with respect to the γ-ray pair so as to produce data as the γ-ray detection positional information. It should be understood that the above-described two detection points imply such positions of one paired radiation detectors 4 whose directions are different from each other at an angle of approximately 180 degrees while the axial center of the hole unit 30 is set as the center.

Next, a description is made of imaging sequential operations executed by both the X-ray CT examination apparatus and the PET-X-ray CT examination apparatus, which are constructed in the above-described manner, with reference to a flow chart of Fig. 8.

First, an X-ray CT image of the examinee 35 is imaged by employing the X-ray CT examination apparatus A (step S50), and then an image reconstructing operation of the data acquired by the X-ray CT examination apparatus A is carried out (step S51). Next, both a PET image and an X-ray CT image as to the examinee 35 are imaged by employing the PET-X-ray CT examination apparatus B (step S52), and then, both the PET data and the X-ray CT data acquired by the PET-X-ray CT examination apparatus B are image-reconstructed so as to acquire both a PET image and an X-ray CT image (step S53). The image reconstructing operation of this X-ray CT image is identical to that of the above-described step S51. As a PET image reconstructing method, in the case of a two-dimensional imaging operation, for example, the above-explained Filtered Back Projection Method may be employed. In the case that a three-dimensional imaging operation is executed, for instance, an image reconstructing operation is carried out by employing, for example, "Fourier ---???" method which is described in IEEE Transactions on Medical Imaging, volume No. 16, page 145, in 1997. As a result, a PET image is acquired. As to the PET image, generation density information of gamma ray pairs is acquired.

It should be understood that the imaging operation by the X-ray CT examination apparatus A has been first of all carried out in this first embodiment. Alternatively, the imaging operation (step S52) by the PET-X-ray CT examination apparatus B may be executed in advance, and thereafter, the image reconstructing operations may be carried out in this order of the steps S53, S50, S51. Also, since the process operations defined in the steps S51 and S53 are carried out by the computer, the process operations defined in the steps S51 and S53 may be alternatively carried out in a parallel manner. Also, as to the imaging order, in such a case that the imaging operation by the PET-X-ray CT examination apparatus B is executed prior to the imaging operation by the X-ray CT examination apparatus A, the process operations defined in the steps S50 and S53 may be carried out in a parallel manner. Also, after both the imaging operations have been accomplished, image reconstructing operations (namely, steps S51 and S53) may be simultaneously carried out, or may be sequentially carried out.

Next, the X-ray CT image acquired from the X-ray CT examination apparatus A is compared with the X-ray CT image acquired from the PET-X-ray CT image by an X-ray CT image positional alignment computer so as to clarify a positional relationship between both these X-ray CT images (step S54).

At this time, both the X-ray CT image acquired in the step S51 and an X-ray CT image which is positionally aligned must be entered into the X-ray CT image positional alignment computer. These X-ray images are inputted by employing, for instance, an internal hospital network so as to transfer data files, or employing a storage medium such as an MO disk. As one example of a method for clarifying a positional relationship between both X-ray CT images, such an image comparing operation may be carried out by employing such shapes which are clearly present in X-ray CT images. For instance, a bone is clearly imaged in an X-ray CT image.

As a result, while a position of the bone is employed as a reference, such information as respective directions of both the X-ray CT images, and rotations and compression/expansion as to the X-ray CT images is acquired. A human body owns various biological portions, for example, from a lung field and the like whose sizes are changed by recipiration, up to a place such as a head portion which does not substantially move. As a consequence, positional relationship clarifying methods must be employed in correspondence with biological portions. For example, in the case of a head portion, since this head portion is not substantially expanded/compressed, such a method for simply rotating, expanding, and compressing images may be satisfactorily employed. In the case of lung fields, there are,personal differences in move directions thereof and move distances thereof. Therefor, there are some cases that X-ray CT images of the lung fields are not matched with each other by merely moving, rotating, enlarging, and compressing these X-ray CT images. In such a case, a non-linear transformation is employed. As an example of this non-linear transformation, there is a deformable model. As an example of the positional alignment by employing the deformable module, the free form deformations may be employed which is described in IEEE Transactions of Medical Imaging, volume No. 18, page 712. This method corresponds to such a method for using a deformable model in which a large movement of an examinee is expressed by employing an affine transformation (transformation by rotation, parallel movement, and enlarge/compression), whereas a local movement is expressed by employing a beta spline function.

Next, while the biological structure image (X-ray CT image acquired by X-ray CT examination apparatus A) is employed which has been positionally aligned, a correcting operation is carried out as to the biological structure image (X-ray CT image acquired by PET-X-ray CT examination apparatus B), the positional relationship of which is known with respect to the biological function image (PET image acquired by PET-X-ray CT examination apparatus B) (step S56).

At this time, in such a case that a coordinate system like a head portion is fixed, a simple interpolation may be carried out. A correcting method is represented by employing Figs. 6A-6E. In Figs. 6A-6E, it is so assumed that a positional relationship between Fig. 6A and Fig. 6C can be grasped since the process operation of the step S54 of Fig. 8 is executed, and furthermore, a positional relationship between Fig. 6A and Fig. 6B can be obtained since the same imaging operation is carried out. In other words, such an assumption is established: points 151a, 151b, 151e, and 151f of an image shown in Fig. 6C correspond to points 150a, 150b, 150e, and 150f of an image shown in Fig. 6A, respectively, and furthermore, since the simultaneous imaging operations are carried out, points 152a, 152b, 152e, and 152f of an image indicated in Fig. 6B correspond to the points 150a, 150b, 150e, and 150f of the image shown in Fig. 6A, respectively. Also, it is so assumed that resolution of the image shown in Fig. 6C is higher than that of the image shown in Fig. 6A.

In this case, for instance, since data of a rectangular shape defined by the points 151a, 151b, 151e, 151f shown in Fig. 6C are incorporated into another rectangular shape defined by the points 150a, 150b, 150e, 150f shown in Fig. 6A, the image can be corrected. This work can be simply carried out by executing an interpolation, even when grids are not matched with each other. The positional relationship between the image of Fig. 6B and the image of Fig. 6D corresponding to the high spatial resolution image of Fig. 6A which has been acquired in the above-described manner can be grasped, so that an image synthesizing operation can be simply carried out. The image of Fig. 6D is synthesized with the image of Fig. 6B by employing this positional relationship, so that an image shown in Fig. 6E can be obtained.

On the other hand, in the case that the non-linear transformation such as a deformable model is employed, mesh sizes do not correspond to each other in such two images as points 150a, 150b, 150e, 150f of Fig. 7A, and points 151a, 151b, 151e, 151f of Fig. 7C. Even in such a case, for example, in accordance with an interpolation method, center points of respective edges are connected to each other, and then, meshes are interpolated in accordance with this line, so that an image can be divided. Since the interpolation is carried out in this manner, an image having high precision can be obtained. As a result, since a process operation similar to that of Fig. 6 is carried out, after a positional relationship between the points 150a, 150b, 150e, 150f of Fig. 7A and the points 151a, 151b, 151e, 151f of Fig. 7C has been obtained, the image shown in Fig. 7A is corrected so as to acquire an image indicated in Fig. 7D. Thereafter, this image of Fig. 7D is synthesized with an image of Fig. 7B, so that such an image indicated in Fig. 7E is obtained.

Then, the acquired images are displayed on a display unit (step S57). As the display method, both the PET image and the X-ray CT image may be displayed in a simultaneous manner, or may be alternatively displayed side by side. As a result, the below-mentioned effect may be achieved.

(1). The precision of the biological structure image which is required so as to grasp the nidus position of the PET image can be obtained by a small amount of data.

It should also be noted that even in such a case that other modalities are employed which indicate such structures as a PET/MRI image and an MRI image which has been separately acquired with respect to the PET/MRI image, these modalities may be alternatively employed instead of the X-ray CT so as to execute the steps of Fig. 8. As the modalities indicative of the structures, there are X-ray CT, MRI, ultrasonic diagnosis, a transmission image of PET, SPTCT image, and the like. Conversely, also in such a case that other biological function diagnostic images such as SPECT and optical topography are employed instead of PET, it is readily possible to conceive that these modalities may be used instead of PET.

(SECOND EMBODIMENT)

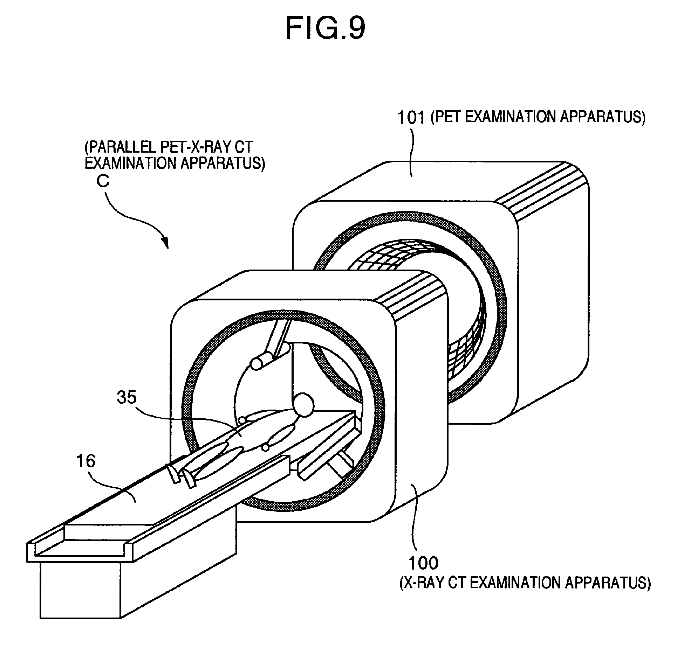

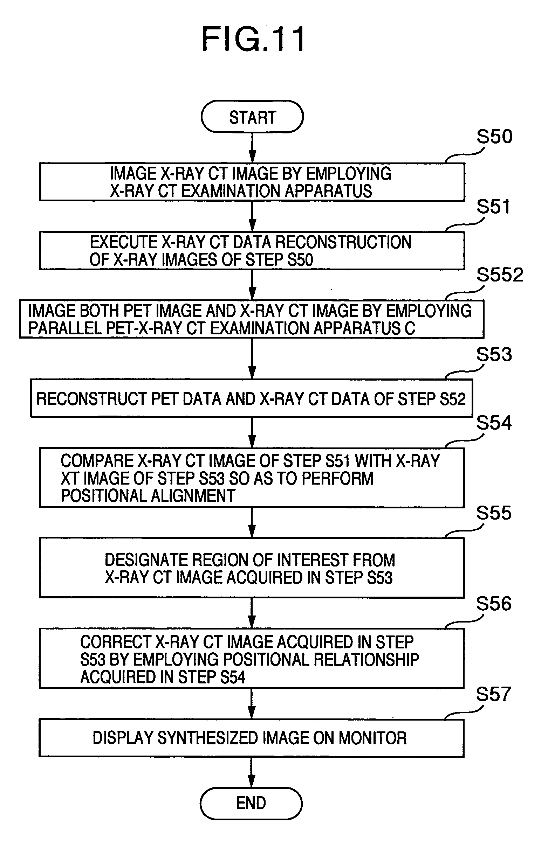

In the first embodiment, the simultaneous imaging operation capable apparatus (PET-X-ray CT examination apparatus B) of both the PET examination apparatus and the X-ray CT examination apparatus has been exemplified. Alternatively, if a positional relationship can be grasped, then simultaneous imaging operations are not necessarily required. To this end, a second embodiment of the present invention in such a case that both a PET examination apparatus and an X-ray CT examination apparatus are arrayed so as to acquire a positional relationship will now be described with reference to Fig. 9 to Fig. 11.

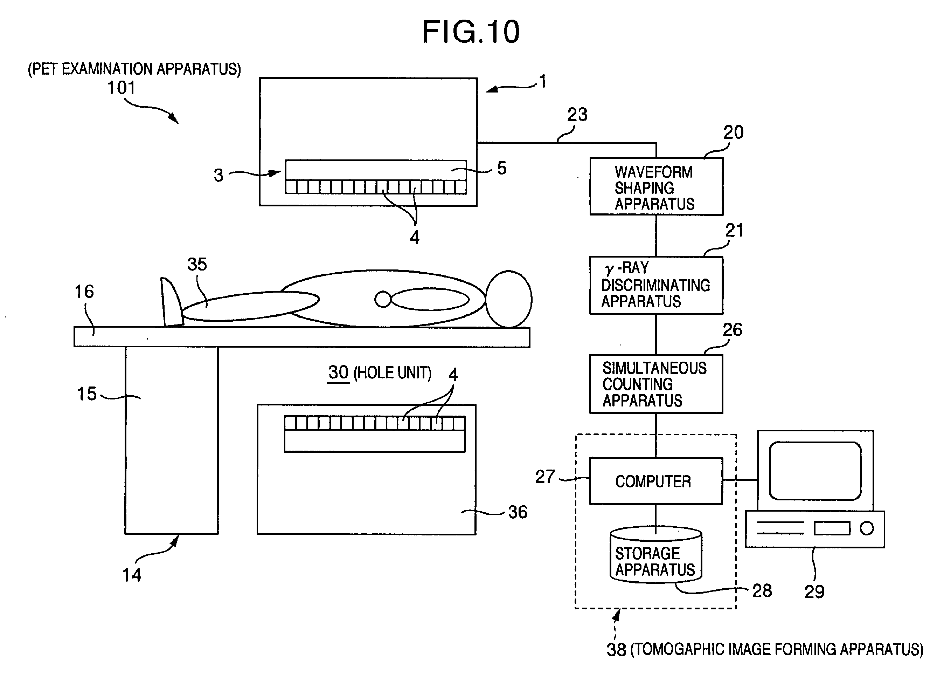

An imaging sequential operation of the second embodiment is not basically different from the imaging sequential operation shown in Fig. 8 of the first embodiment. Instead of the process operation defined in the step S52 of the first embodiment in which the imaging operations by the PET examination apparatus and the X-ray CT examination apparatus are carried out in the simultaneous manner, an imaging operation is carried out in a parallel PET-X-ray CT examination apparatus "C" capable of grasping a positional relationship. A flow chart in which this step S52 has been changed into a step S552 is indicated in Fig. 10.

First, the parallel PET-X-ray CT examination apparatus "C" used in the step S552 is explained with reference to Fig. 9. In the parallel PET-X-ray CT examination apparatus "C", both an X-ray CT examination apparatus 100 and a PET examination apparatus 101 are arranged in a parallel manner. A positional relationship between the X-ray CT examination apparatus 100 and the PET examination apparatus 101 is known, and a positional alignment between these examination apparatuses 100 and 101 can be readily performed. An imaging method of the X-ray CT examination apparatus 100 is similar to that of the X-ray CT examination apparatus "A" described in the first embodiment.

Thus, the PET examination apparatus 101 will now be described with reference to Fig. 10. It should be understood that the same reference numerals shown in the above-described first embodiment will be employed as those for denoting the same, or similar structural elements of the second embodiment, and explanations thereof are omitted.

While the PET imaging apparatus 101 is installed along a direction perpendicular to a longitudinal direction of a bed 16, a radiation detector ring-shaped member 3 is installed in a casing 36. The radiation detector ring-shaped member 3 contains a ring-shaped holding unit 5, and a large number of radiation detectors 4 which are installed in a ring shape inside the ring-shaped holding unit 5. A hole unit 30 is formed inside the radiation detectors 4 of the radiation detector ring-shaped member 3. The hole unit 30 is made of a through hole, and corresponds to an observation region into which the bed 16 is inserted. A large number of radiation detectors 4 (approximately 10,000 pieces in total) are arranged in the form of plural arrays in such a manner that these radiation detectors 4 are installed on the ring-shaped holding unit 5 not only along the circumferential direction, but also along the axial direction of the hole unit 30. The radiation detector 4 corresponds to a semiconductor radiation detector, while a semiconductor element portion thereof is constructed of CdTe (cadmium tellurium) which is made of a cube sized by 5 mm X 5 mm and corresponds to a detecting portion thereof. Alternatively, this element portion may be constituted by either GaAs (gallium arsenide) or CZT (cadmium zinc tellurium).

The respective radiation detectors 4 are connected to respective waveform shaping apparatuses 20 corresponding thereto. These waveform shaping apparatuses 20 are provided one by one with respect to each of the radiation detectors 4. The waveform shaping apparatuses 20 are connected to the respective radiation detectors 4 and γ-ray discriminating apparatuses 21. The γ-ray discriminating apparatus 21 is connected via a simultaneous counting apparatus 26 to a computer 27. A total number of the simultaneous counting apparatus 26 is 1, and is connected to all of the γ-ray discriminating apparatuses 21. Both a storage apparatus 28 and a display apparatus 29 are connected to the computer 27.

A concrete PET examination, which is executed by the PET imaging apparatus 101, will now be described. First, a chemical PET drug is injected into the examinee 35 in order that injected body interior radiation becomes equal to 370 MBq. The chemical PET drug is selected in accordance with a purpose of examination (place of cancer is grasped, cardiac arteria stream is examined, etc.). After the chemical PET drug has been injected and a predetermine time period has elapsed, while the examinee 35 is laid on the bed 16, this examinee 35 is inserted into the hole unit 30.

Gamma rays of 511 KeV which are caused by the chemical PET drug are being emitted from the examinee 35 located within the hole unit 30. The radiation detectors 4 other than the first radiation detectors 4 detect these gamma rays emitted from the examinee 35, and output detection signals of these gamma rays (will be referred to as "γ-ray detection signals" hereinafter). The γ-ray detection signals are transferred via the relevant wiring lines 23 and the corresponding waveform shaping apparatuses 20 to the γ-ray discriminating apparatuses 21. A large number of γ rays which have been generated within the interior portion of the examinee 35 due to the chemical PET drug are not emitted along a specific direction, but are emitted along a universal direction. As previously explained, these γ rays are emitted in such a condition that these γ rays constitute paired γ rays, and the paired γ rays are emitted along directions substantially opposite to each other (180 degrees ± 0.6 degrees), and then, are detected by any of the radiation detectors 4 of the radiation detector ring-shaped member 3.

The γ-ray detection signals detectors 4 are entered into the waveform shaping apparatuses 20. The waveform shaping apparatuses 20 shape the waveforms obtained in the radiation detectors 4 so as to produce such waveforms which can be read by the γ-ray discriminating apparatus 21. The output signals of the waveform shaping apparatus 20 are supplied to the γ-ray discriminating apparatuses 21. Next, a role of this γ-ray discriminating apparatus 21 will now be described. As previously explained, energy of such γ rays is equal to 511 KeV, which are produced in the interior body of the examinee 35, since positrons emitted from the chemical PET drug are annihilated. However, all of the energy of the γ rays within the semiconductor element portion are not always changed into electrons. On the other hand, in order to eliminate noise which is caused by low-energy gamma rays which have been scattered in the interior body of the examinee 35, only such γ-ray detection signals which are approximated to 511 KeV are required as data. As a consequence, while the γ-ray discriminating apparatus 21 defines energy of e.g., 450 KeV lower than 511 KeV as an energy setting value, when an imaging signal having energy higher than, equal to this energy setting value (will be referred to as "first energy setting value") is inputted into the γ-ray discriminating apparatus 21, this γ-rays discriminating apparatus 21 generates a pulse signal having predetermined energy. In other words, the γ-ray discriminating apparatus 21 corresponds to such an apparatus for generating the pulse signal having the above-described energy when such an imaging signal (namely, γ-ray detection signal) having energy higher than, or equal to the first energy setting value is inputted thereinto.

As previously explained, in order that a γ-ray detection signal having specific energy is processed in the γ-ray discriminating apparatus 21, such a filter capable of penetrating therethrough imaging signals having a predetermined energy range may be alternatively provided within the γ-ray discriminating apparatus 21 (otherwise, prestage of γ-ray discriminating apparatus 21). The γ-ray discriminating apparatus 21 generates a pulse signal with respect to an imaging signal which has passed a first filter.

The simultaneous counting apparatus 26 performs a simultaneous counting operation every γ-ray pair in such a manner that the pulse signals outputted from the γ-ray discriminating apparatuses 21 are entered into this simultaneous counting apparatus 26, and these inputted pulse signals are employed, and then, calculates a count value with respect to the γ-ray detection signal. Furthermore, the simultaneous counting apparatus 26 processes two detection points where each of γ-rays of a γ-ray pair have been detected based upon one pair of pulse signals with respect to the γ-ray pair so as to produce data as the γ-ray detection positional information. It should be understood that the above-described two detection points imply such positions of one paired radiation detectors 4 whose directions are different from each other at an angle of approximately 180 degrees while the axial center of the hole unit 30 is set as the center. The positional information outputted from the simultaneous counting apparatus 26 is stored in the storage apparatus 28.

In the parallel PET-X-ray CT examination apparatus C constructed in accordance with the above-described manner, both a PET image and an X-ray CT image are acquired in a step S552. Next, the data obtained in the parallel PET-X-ray CT examination apparatus C is image-reconstructed so as to obtain a PET image and a CT image (step S53). The image reconstructing operation of this second embodiment is identical to that of the first embodiment.

Subsequently, a positional alignment may be carried out in a similar manner to that of the first embodiment. However, a positional relationship between the PET examination apparatus and the X-ray CT examination apparatus cannot be completely grasped. As a consequence, in this second embodiment, in a step S55, a positional relationship between the PET image and the X-ray CT image is acquired from the positional relationship between the PET examination apparatus and the X-ray CT examination apparatus. For instance, assuming now that centers of these PET and X-ray CT examination apparatus are separated by an X cm, when the bed 16 is moved over a distance of X cm, the positions are matched with each other. The positional alignment is carried out by utilizing this technical idea. If only the positional relationship between the PET image and the X-ray CT image can be grasped, then the succeeding process operation may be carried out in a similar manner to that of the first embodiment.

As a consequence, in addition to the above-described effect (1) achieved in the first embodiment, the below-mentioned effect may be furthermore achieved.

(2). Even in such a case that the PET-X-ray CT examination apparatus B capable of performing the simultaneous imaging operations, and further, only such an X-ray CT examination apparatus having low resolution is located in the vicinity of a PET examination apparatus, the positional alignment can be carried out with respect to an X-ray CT examination apparatus having high spatial resolution.

(THIRD EMBODIMENT)

Although the positional alignments have been carried out in the images in the first and second embodiments, this third embodiment describes such a method for synthesizing sinogram data preceding to the above-explained images, and images in a frequency space.

These positional alignments may become very effective in the case that there are different filters when image reconstructing operations of apparatus are carried out. This is because images themselves are different from each other in such a case that various sorts of filters are employed when an image reconstructing operation is carried out, and/or in such a case that different image reconstructing methods are employed. As a consequence, it is very important that a positional alignment is carried out by employing a sinogram. This third embodiment will be described as follows:

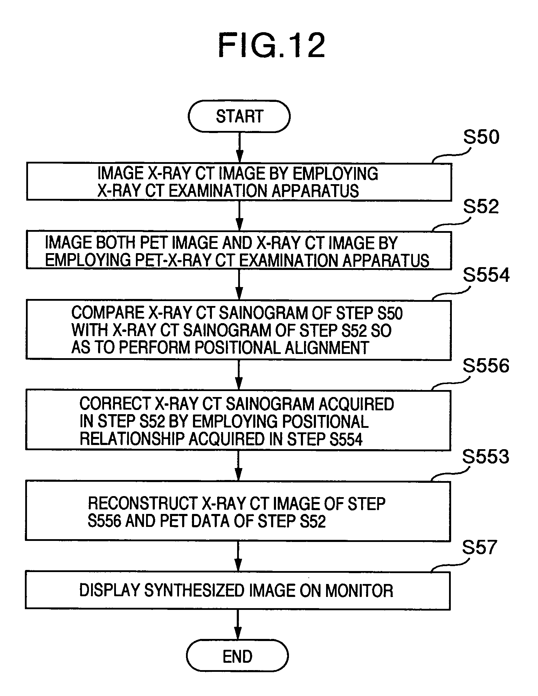

Fig. 12 is a flow chart for describing both an imaging operation and an image synthesizing operation, according to this third embodiment. First, an imaging method is carried out in the same manner to that of the first embodiment and the second embodiment. Assuming now that an imaging operation according to this third embodiment is carried out in a similar manner to that of the first embodiment, the process operations defined in the step S50 and the step S52 are executed.

Next, the X-ray CT sinogram acquired in the step S50 is compared with the X-ray CT sinogram acquired in the step S52 so as to perform a positional alignment (step S554). Data acquired before an image reconstructing operation is referred to as a sinogram, and corresponds to data in an r-θ space. Since information as to each voxel in a sinogram is expressed by a curve, both a featured curve contained in the X-ray CT data acquired in the step S50 and a featured curve contained in the X-ray CT data acquired in the step S52 are extracted, and then, a positional alignment is carried out in such a manner that the positions of these featured curves are overlapped to each other. As to such a sinogram from which a higher resolution image can be acquired, dividing numbers of data along the r direction and the θ direction are larger, as compared with another sinogram from which a lower resolution image is acquired. As a result, similar to Fig. 8 of the first embodiment, data of low resolution CT is corrected based upon data of high resolution CT (step S556). Thereafter, the corrected X-ray CT data of the PET-X-ray CT and the PET data thereof are image-reconstructed (step S553). The positional relationship between these X-ray CT and PET data obtained after the image reconstruction is already known since the simultaneous imaging operation is carried out, so that this positional relationship has been obtained. Accordingly, an image synthesizing operation is carried out by employing the known positional relationship (step S57).

In this third embodiment, the positional aligning method on the sinogram has been disclosed. Alternatively, the positional aligning method may be carried out even in such a frequency space that the sinogram data is Fourier-transformed. As a result, the below-mentioned merit can be obtained in addition to the merits achieved in the first embodiment and the second embodiment.

(3). Since the positional alignment can be carried out based upon the data which have not been filtered, these data can be applied even between apparatuses, the filter functions of which are different from each other. Since the correction is performed before the image reconstructing operation is carried out, the images which have been corrected in the high precision as the transmission data can be used when the images are reconstructed in the step S553. Normally, in a PET examination, an absorption correction is carried out when an image reconstructing operation is performed. At this time, since an image of high resolution CT is used, correction precision may be improved.

(FOURTH EMBODIMENT)

In the first embodiment, the interpolating operations have been carried out in all of the regions. However, normally speaking, a region of interest is present only in a portion of an image. As a consequence, since only a portion of an image is processed in high precision and the remaining portion of this image is processed in low precision, the image may be compressed. In this fourth embodiment, while this technical idea is utilized, such a method for processing only a necessary image portion in high precision is represented.

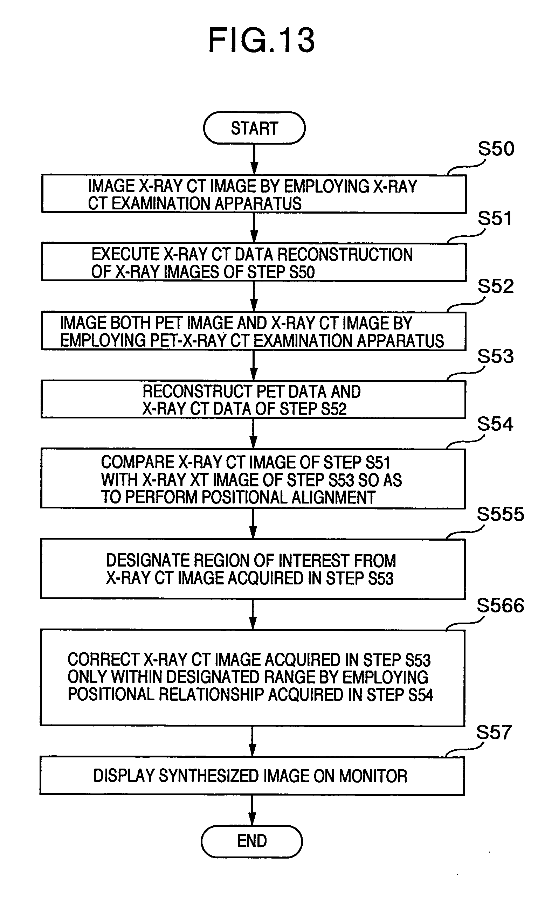

Fig. 13 is a flow chart for describing an imaging operation of this fourth embodiment. In this flow chart, steps defined from S50 to S53 are similar to those of the first embodiment. Next, a region of interest by a user is set by employing the X-ray CT image acquired in the step S53 (step S555). As a method for extracting a region of interest, the following methods may be conceived: a manual extracting method, an automatic extracting method, and a semi-automatic extracting method. When a region of interest is manually extracted, the user performs a marking operation on the region of interest of the X-ray CT image. For example, the user surrounds the region of interest on a console screen by operating an input apparatus such as a mouse. A computer determines the region of interest based upon the input data from the user. On the other hand, in the case that a region of interest is automatically extracted, the user selects the region of interest, or previously informs the region of interest to the computer. For instance, in such a case that the region of interest selected by the user is a liver, the computer extracts a liver region. As an extracting method, there is the following extracting method. That is, for example, since a range of CT values of a liver has been previously determined, the computer applies a threshold value to an X-ray CT image so as to extract the liver. Also, as the semi-automatic extracting method, there is a region growing method. In this region growing method, since the user marks one point within a region of interest, the computer automatically calculates such a region which is approximated to the marking CT value so as to determine the region of interest. In both the automatic extracting method and the semi-automatic extracting method, the computer requests the user to confirm as to whether or not the region is correctly extracted in order to avoid a misdiagnosis.

Next, the correcting operation executed in the step S56 of the first embodiment is carried out as to only the extracted portion (step S566). Since the correcting method is similar to that of the first embodiment, explanations thereof are omitted. Then, thereafter, an image is displayed in a step S57. As a result, the below-mentioned effect can be furthermore achieved in addition to the effects obtained in the first to third embodiments.

(4). Since the high resolution image of only the necessary region can be obtained, the file size can be reduced.

It should be further understood by those skilled in the art that although the foregoing description has been made on embodiments of the invention, the invention is not limited thereto and various changes and modifications may be made without departing from the spirit of the invention and the scope of the appended claims.

**Claims**

1. A nidus position specifying system for specifying a nidus position during a biological function examination, comprising:

   a biological function examination apparatus (PET-X-ray CT examination apparatus "B");
   a first biological structure examination apparatus (PET-X-ray CT examination apparatus "B") for imaging a biological structure image, the positional relationship of which is known with respect to a biological function image which has been imaged by said biological function apparatus; and
   a second biological structure examination apparatus (X-ray CT examination apparatus "A") for imaging another biological structure image different from said biological structure image whose positional relationship is known; wherein:

   said nidus position specifying system specifies a position of said biological structure image imaged by said second biological structure examination apparatus by employing said known positional relationship and a positional relationship between the biological structure image imaged by said first biological structure examination apparatus, and the biological structure image imaged by said second biological structure examination apparatus.

2. A nidus position specifying system for specifying a nidus position during a biological function examination, comprising:

   a biological function examination apparatus (PET-X-ray CT examination apparatus "B");
   a first biological structure examination apparatus (PET-X-ray CT examination apparatus "B") for imaging a biological structure image, the positional relationship of which is known with respect to a biological function image which has been imaged by said biological function apparatus; and
   a second biological structure examination apparatus (X-ray CT examination apparatus "A") for imaging another biological structure image different from said biological structure image whose positional relationship is known; wherein:

   said nidus position specifying system corrects the biological structure image whose positional relationship is known with respect to the biological function image imaged by said biological function examination apparatus by employing a positional relationship between the biological structure image imaged by said first biological structure examination apparatus, and the biological structure image imaged by said second biological structure examination apparatus.

3. A nidus position specifying system for specifying a nidus position during a biological function examination, comprising:

   a biological function examination apparatus (PET-X-ray CT examination apparatus "B");
   a first biological structure examination apparatus (PET-X-ray CT examination apparatus "B") for imaging a biological structure image, the positional relationship of which is known with respect to a biological function image which has been imaged by said biological function apparatus; and
   a second biological structure examination apparatus (X-ray CT examination apparatus "A") for imaging another biological structure image different from said biological structure image whose positional relationship is known; wherein:

   said nidus position specifying system corrects an arbitrary portion of the biological structure image whose positional relationship is known with respect to the biological function image imaged by said biological function examination apparatus by employing a positional relationship between the biological structure image imaged by said first biological structure examination apparatus, and the biological structure image imaged by said second biological structure examination apparatus.

4. A nidus position specifying system as claimed in one of claims 1 to 3, wherein:

a stage for correcting said biological structure image corresponds to a real space or a frequency space.

5. A nidus position specifying system as claimed in one of claims 1 to 3, wherein:

said biological function examination apparatus corresponds to a PET examination apparatus.

6. A nidus position specifying system as claimed in one of claims 1 to 3 wherein:

said biological function examination apparatus corresponds to a SPECT examination apparatus.

7. A nidus position specifying system as claimed in one of claims 1 to 3, wherein:

at least any one of said first biological structure examination apparatus and said second biological structure examination apparatus corresponds to an MRI examination apparatus.

8. A nidus position specifying system as claimed in one of claims 1 to 3, wherein:

at least any one of said first biological structure examination apparatus and said second biological structure examination apparatus corresponds to an X-ray CT examination apparatus.

9. A radiation examination apparatus comprising: a nidus position specifying system for specifying a nidus position during a biological function examination, equipped with:

a biological function examination apparatus (PET-X-ray CT examination apparatus "B"); a first biological structure examination apparatus (PET-X-ray CT examination apparatus "B") for imaging a biological structure image, the positional relationship of which is known with respect to a biological function image which has been imaged by said biological function apparatus; and

a second biological structure examination apparatus (X-ray CT examination apparatus "A") for imaging another biological structure image different from said biological structure image whose positional relationship is known; in which said nidus position specifying system specifies a position of said biological structure image imaged by said second biological structure examination apparatus by employing said known positional relationship and a positional relationship between the biological structure image imaged by said first biological structure examination apparatus, and the biological structure image imaged by said second biological structure examination apparatus.

10. A radiation examination apparatus comprising:

a nidus position specifying system for specifying a nidus position during a biological function examination, equipped with:

a biological function examination apparatus (PET-X-ray CT examination apparatus "B"); a first biological structure examination apparatus (PET-X-ray CT examination apparatus "B") for imaging a biological structure image, the positional relationship of which is known with respect to a biological function image which has been imaged by said biological function apparatus; and a second biological structure examination apparatus (X-ray CT examination apparatus "A") for imaging another biological structure image different from said biological structure image whose positional relationship is known; in which said nidus position specifying system corrects the biological structure image whose positional relationship is known with respect to the biological function image imaged by said biological function examination apparatus by employing a positional relationship between the biological structure image imaged by said first biological structure examination apparatus, and the biological structure image imaged by said second biological structure examination apparatus.

11. A radiation examination apparatus comprising:

a nidus position specifying system for specifying a nidus position during a biological function examination, equipped with:

a biological function examination apparatus (PET-X-ray CT examination apparatus "B");

a first biological structure examination apparatus (PET-X-ray CT examination apparatus "B") for imaging a biological structure image, the positional relationship of which is known with respect to a biological function image which has been imaged by said biological function apparatus; and

a second biological structure examination apparatus (X-ray CT examination apparatus "A") for imaging another biological structure image different from said biological structure image whose positional relationship is known; in which said nidus position specifying system corrects an arbitrary portion of the biological structure image whose positional relationship is known with respect to the biological function image imaged by said biological function examination apparatus by employing a positional relationship between the biological structure image imaged by said first biological structure examination apparatus, and the biological structure image imaged by said second biological structure examination apparatus.

# FIG.1

A (X-RAY CT EXAMINATION APPARATUS)

1a (X-RAY CT IMAGING APPARATUS)

18a   17a   13a

9a

7a
(X-RAY SOURCE CIRCUMFERENTIAL
DIRECTION TRANSPORTING APPARATUS )

34

35 (EXAMINEE)

16a

15a

30a (HOLE UNIT)   32

80

23a

36a   31

14a

SIGNAL
PROCESSING
APPARATUS

22a

COMPUTER

27a

STORAGE
APPARATUS

28a

29a

38a (TOMOGRAPHIC IMAGE FORMING APPARATUS)

EP 1 506 742 A1

# FIG.2

(X-RAY SOURCE)

34  9a  30a

16

35

36a

6a  32
(COLLIMATOR)

31

80 (RADIATION DETECTOR)

# FIG.3

17 (DRIVING CONTROL APPARATUS)

18 (X-RAY SOURCE CONTROL APPARATUS)

3 (RADIATION DETECTOR RING-SHAPED MEMBER)

B (PET-X-RAY CT EXAMINATION APPARATUS)

10

12

8
(X-RAY SOURCE
APPARATUS)

10A

11

9
(X-RAY SOURCE)

35 (EXAMINEE)

16 (BED)

15

14

30 (HOLE UNIT)

SIGNAL DISCRIMINATING APPARATUS

19

37
(SIGNAL PROCESSING MEANS)

26

SIMULTANEOUS COUNTING APPARATUS

27

COMPUTER

5

4

STORAGE APPARATUS

28

29

38 (TOMOGTAPHIC IMAGE FORMING APPARATUS)

1 (IMAGING APPARATUS)

36 (CASING)

EP 1 506 742 A1

# FIG.4

1 (IMAGING APPARATUS)

3

9

(EXAMINEE)
35

5

16

30 (HOLE UNIT)

4    6 (SUPPORTING MEMBER)

# FIG.5

# FIG.6A

150a  150b

150e

150f

# FIG.6B

152a  152b

152e

152f

# FIG.6C

151a  151b

151e

151f

# FIG.6D

150a  150b

150e

150f

# FIG.6E

# FIG.7A

150a    150b

150e

150f

# FIG.7B

152a    152b

152e

152f

# FIG.7C

151a    151b

151e

151f

# FIG.7D

150a    150b

150e

150f

# FIG.7E

# FIG.8

START

S50
IMAGE X-RAY CT IMAGE BY EMPLOYING X-RAY
CT EXAMINATION APPARATUS A

S51
EXECUTE X-RAY CT DATA RECONSTRUCTION
OF X-RAY IMAGES OF STEP S50

S52
IMAGE BOTH PET IMAGE AND X-RAY CT IMAGE BY
EMPLOYING PET-X-RAY CT EXAMINATION APPARATUS B

S53
RECONSTRUCT PET DATA AND X-RAY CT DATA OF STEP S52

S54
COMPARE X-RAY CT IMAGE OF STEP S51 WITH
X-RAY XT IMAGE OF STEP S53 SO AS TO PERFORM
POSITIONAL ALIGNMENT

S56
CORRECT X-RAY CT IMAGE ACQUIRED IN STEP
S53 BY EMPLOYING POSITIONAL RELATIONSHIP
ACQUIRED IN STEP S54

S57
DISPLAY SYNTHESIZED IMAGE ON MONITOR

END

# FIG.9

101 (PET EXAMINATION APPARATUS)

(PARALLEL PET-X-RAY CT
EXAMINATION APPARATUS)
C

35

16

100
(X-RAY CT EXAMINATION APPARATUS)

# FIG.10

(PET EXAMINATION APPARATUS)
101

1

23

20
WAVEFORM SHAPING APPARATUS

3

5

4

21
γ-RAY DISCRIMINATING APPARATUS

35

16

15

14

26
SIMULTANEOUS COUNTING APPARATUS

30 (HOLE UNIT)
4

36

27
COMPUTER

28
STORAGE APPARATUS

29

38 (TOMOGAPHIC IMAGE FORMING APPARATUS)

EP 1 506 742 A1

# FIG.11

```
                    ┌─────────┐
                    │  START  │
                    └────┬────┘
                         │                                    S50
        ┌────────────────▼─────────────────┐
        │  IMAGE X-RAY CT IMAGE BY EMPLOYING│
        │  X-RAY CT EXAMINATION APPARATUS   │
        └────────────────┬─────────────────┘
                         │                                    S51
        ┌────────────────▼─────────────────┐
        │  EXECUTE X-RAY CT DATA RECONSTRUCTION │
        │  OF X-RAY IMAGES OF STEP S50      │
        └────────────────┬─────────────────┘
                         │                                    S552
    ┌────────────────────▼──────────────────────────┐
    │ IMAGE BOTH PET IMAGE AND X-RAY CT IMAGE BY EMPLOYING│
    │ PARALLEL PET-X-RAY CT EXAMINATION APPARATUS C  │
    └────────────────────┬──────────────────────────┘
                         │                                    S53
      ┌──────────────────▼────────────────────────┐
      │ RECONSTRUCT PET DATA AND X-RAY CT DATA OF STEP S52│
      └──────────────────┬────────────────────────┘
                         │                                    S54
        ┌────────────────▼─────────────────┐
        │ COMPARE X-RAY CT IMAGE OF STEP S51 WITH X-RAY│
        │ XT IMAGE OF STEP S53 SO AS TO PERFORM │
        │ POSITIONAL ALIGNMENT              │
        └────────────────┬─────────────────┘
                         │                                    S55
        ┌────────────────▼─────────────────┐
        │ DESIGNATE REGION OF INTEREST FROM │
        │ X-RAY CT IMAGE ACQUIRED IN STEP S53│
        └────────────────┬─────────────────┘
                         │                                    S56
        ┌────────────────▼─────────────────┐
        │ CORRECT X-RAY CT IMAGE ACQUIRED IN STEP│
        │ S53 BY EMPLOYING POSITIONAL RELATIONSHIP│
        │ ACQUIRED IN STEP S54             │
        └────────────────┬─────────────────┘
                         │                                    S57
        ┌────────────────▼─────────────────┐
        │ DISPLAY SYNTHESIZED IMAGE ON MONITOR│
        └────────────────┬─────────────────┘
                         │
                    ┌────▼────┐
                    │   END   │
                    └─────────┘
```

# FIG.12

START

IMAGE X-RAY CT IMAGE BY EMPLOYING
X-RAY CT EXAMINATION APPARATUS

S50

IMAGE BOTH PET IMAGE AND X-RAY CT IMAGE BY
EMPLOYING PET-X-RAY CT EXAMINATION APPARATUS

S52

COMPARE X-RAY CT SAINOGRAM OF STEP S50
WITH X-RAY CT SAINOGRAM OF STEP S52 SO
AS TO PERFORM POSITIONAL ALIGNMENT

S554

CORRECT X-RAY CT SAINOGRAM ACQUIRED
IN STEP S52 BY EMPLOYING POSITIONAL
RELATIONSHIP ACQUIRED IN STEP S554

S556

RECONSTRUCT X-RAY CT IMAGE OF STEP
S556 AND PET DATA OF STEP S52

S553

DISPLAY SYNTHESIZED IMAGE ON MONITOR

S57

END

# FIG.13

START

S50
IMAGE X-RAY CT IMAGE BY EMPLOYING X-RAY
CT EXAMINATION APPARATUS

S51
EXECUTE X-RAY CT DATA RECONSTRUCTION
OF X-RAY IMAGES OF STEP S50

S52
IMAGE BOTH PET IMAGE AND X-RAY CT IMAGE BY
EMPLOYING PET-X-RAY CT EXAMINATION APPARATUS

S53
RECONSTRUCT PET DATA AND
X-RAY CT DATA OF STEP S52

S54
COMPARE X-RAY CT IMAGE OF STEP S51
WITH X-RAY XT IMAGE OF STEP S53 SO AS
TO PERFORM POSITIONAL ALIGNMENT

S555
DESIGNATE REGION OF INTEREST FROM
X-RAY CT IMAGE ACQUIRED IN STEP S53

S566
CORRECT X-RAY CT IMAGE ACQUIRED IN STEP S53
ONLY WITHIN DESIGNATED RANGE BY EMPLOYING
POSITIONAL RELATIONSHIP ACQUIRED IN STEP S54

S57
DISPLAY SYNTHESIZED IMAGE ON MONITOR

END

## EUROPEAN SEARCH REPORT

| | European Patent Office | | Application Number |
|---|---|---|---|
| | | | EP 04 01 9058 |

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 6 019 724 A (GRONNINGSAETER AAGE ET AL) 1 February 2000 (2000-02-01)<br>* abstract; figures 16A-C *<br>* column 4, line 65 - column 5, line 4 *<br>* column 10, lines 5-31 * | 1-3,7-11 | A61B6/03<br>G06T5/50 |
| X | WO 00/67202 A (LABARRE JEAN ; STEFANI ERIC (FR); GE MEDICAL SYST SA (FR); KNOPLIOCH J) 9 November 2000 (2000-11-09)<br>* abstract *<br>* page 4, line 14 - page 5, line 9 *<br>* page 5, line 24 - page 6, line 10; claims 1,10 * | 1-3,7-11 | |
| A | FÖRSTER G J ET AL.: "SPET/CT image co-registration in the abdomen with a simple and cost-effective tool"<br>EUROPEAN JOURNAL OF NUCLEAR MEDICINE AND MOLECULAR IMAGING,<br>vol. 30, no. 1,<br>1 January 2003 (2003-01-01), pages 32-39, XP002306546<br>* the whole document * | 6 | TECHNICAL FIELDS SEARCHED (Int.Cl.7)<br><br>A61B<br>G06T |
| A | TARANTOLA G ET AL.: "PET instrumentation and reconstruction algorithms in whole-body applications"<br>THE JOURNAL OF NUCLEAR MEDICINE,<br>vol. 44, no. 5, 1 May 2003 (2003-05-01), pages 756-769, XP002306547<br>* the whole document * | 5 | |

The present search report has been drawn up for all claims

| Place of search | Date of completion of the search | Examiner |
|---|---|---|
| Munich | 23 November 2004 | Lahorte, P |

EPO FORM 1503 03.82 (P04C01)

**ANNEX TO THE EUROPEAN SEARCH REPORT**
**ON EUROPEAN PATENT APPLICATION NO.** EP 04 01 9058

This annex lists the patent family members relating to the patent documents cited in the above-mentioned European search report.
The members are as contained in the European Patent Office EDP file on
The European Patent Office is in no way liable for these particulars which are merely given for the purpose of information.

23-11-2004

| Patent document cited in search report | | Publication date | Patent family member(s) | | Publication date |
|---|---|---|---|---|---|
| US 6019724 | A | 01-02-2000 | WO<br>AU<br>WO | 9625882 A1<br>4851396 A<br>9625881 A1 | 29-08-1996<br>11-09-1996<br>29-08-1996 |
| WO 0067202 | A | 09-11-2000 | FR<br>EP<br>WO<br>JP | 2793055 A1<br>1092207 A1<br>0067202 A1<br>2002542915 T | 03-11-2000<br>18-04-2001<br>09-11-2000<br>17-12-2002 |

EPO FORM P0459

For more details about this annex : see Official Journal of the European Patent Office, No. 12/82